# EUROPEAN PATENT SPECIFICATION

(11) **EP 3 570 899 B1**
(45) Date of publication and mention of the grant of the patent: **10.03.2021**
(21) Application number: 18703146.3
(22) Date of filing: 18.01.2018
(51) Int. Cl.: A61L 29/08

(54) **METHODS OF STERILIZING A HYDROPHILICALLY COATED MEDICAL DEVICE**
VERFAHREN ZUR STERILISATION EINER HYDROPHIL BESCHICHTETEN MEDIZINISCHEN VORRICHTUNG
PROCÉDÉS DE STÉRILISATION D'UN DISPOSITIF MÉDICAL À REVÊTEMENT HYDROPHILE

(30) Priority: 20.01.2017 US 201762448708 P
(43) Date of publication of application: 27.11.2019
(60) Divisional application: 21151601.8
(73) Proprietor: Hollister Incorporated, Libertyville, IL 60048 (US)
(72) Inventor: MONTES DE OCA, Horacio, Libertyville IL 60048 (US); HENRY, Jerome A., Libertyville IL 60048 (US); ROSTAMI, Shamsedin, Libertyville IL 60048 (US)
(74) Representative: FRKelly
(86) International application number: PCT/US2018/014233
(87) International publication number: WO 2018/136630

(56) References cited:
- WO-A1-2007/137699
- WO-A1-2013/017547
- WO-A1-2016/168461

## Description

### Related Application

The present application claims the benefit and priority of U.S.

Provisional Patent Application No. 62/448,708, filed January 20, 2017.

### TECHNICAL FIELD

The present disclosure generally relates to methods of radiation sterilizing a hydrophilically coated medical device in a hydration environment, and more particularly, to methods for radiation sterilization of hydrophilic coatings in a hydration environment that include applying a protective coating over the hydrophilic coating prior to exposing the hydrophilic coating to sterilizing radiation.

### BACKGROUND

It is known to coat medical devices, such as urinary catheters, with a hydrophilic coating. When the hydrophilic coating is wetted or hydrated with a wetting fluid, such as water, it becomes extremely lubricous which eases introduction of the device into the body and aids in reducing pain and discomfort associated with such introduction.

In some applications, the hydrophilically coated medical device is provided in a "dry" state wherein the user is required to wet the hydrophilic coating with a wetting fluid immediately prior to insertion into the body. In other applications, it is desirable to provide a hydrophilically coated medical device that is in a ready-to-use condition right out of the package. In the field of urinary catheters, a hydrophilically coated catheter may be provided in a catheter package that has a hydration environment that hydrates/wets the catheter. In one type of catheter assembly, the hydrophilically coated catheter is stored in the package in contact with liquid water so that the hydrophilic coating is wetted within the package and the catheter is ready for use right out of the package. In other catheter package assemblies, the hydrophilically coated catheter may be provided in a catheter package that has a vapor hydration atmosphere wherein the hydrophilic coating is wetted by water vapor so that the catheter is ready to use right out of package for the end user.

For various reasons, including but not limited to efficiency, effectiveness and cost, it is desirable to radiation sterilize packaged medical device assemblies. In some instances, the hydrophilically coated medical device and liquid water are placed in the package and the package is sealed. The liquid water wets/hydrates the hydrophilic coating by being in direct contact with the hydrophilic coating or the water may provide a water vapor which wets/hydrates the hydrophilic coating. After the package is sealed, the package having the hydrophilically coated medical device and water (liquid and/or vapor) therein is exposed to radiation, such as gamma or E-Beam radiation, to sterilize the medical device. It has been found, however, that sterilization of hydrophilic coatings in the hydrated state or while in contact with a wetting fluid can result in degradation of the coating or excessive crosslinking that can lead to an increase of coefficient of friction (decrease in lubricity) of the coating and/or cause instability of the coating which may result in the coating undesirably detaching from the medical device prior to or during use.

WO 2007/137699 discloses an hydrophilic (PVP) coating. It was found that it is possible to maintain or improve dry-out time and/or lubricity of a hydrophilic coating sterilised by irradiation in the presence of water by carrying out the sterilisation in the presence of a specific stabilizing agent, such as tetraethylenglycol, propylenglycol or glycerol. The stabilising compound is generally present in the coating itself but a presence in the aqueous wetting fluid may be more effective. Conveniently, the device is sterilised while being in a sealed packaging, together with the wetting fluid.

Therefore, there remains a need for methods of sterilizing medical devices having hydrophilic coatings.

### SUMMARY

In one aspect, a method of sterilizing a medical device that includes a hydrophilic surface according to claims 1-8.

In another aspect, a hydrophilically coated medical assembly that includes a package containing a medical device having a hydrophilic coating thereon and a protective coating over the hydrophilic coating according to claims 9-14.

### DESCRIPTION

The present disclosure relates to methods for sterilizing medical devices having a hydrophilic surface, such as a medical device having a hydrophilic coating on a surface of the medical device. Such methods include applying a protective coating over the hydrophilic surface of the medical device, placing the medical device into a hydration environment, and then exposing the medical device to a sufficient amount of sterilization radiation, such as gamma or E-Beam radiation. The hydration environment may be an environment wherein the medical device is in direct contact with liquid water and/or wherein the medical device is exposed to water vapor.

The protective coating temporarily prevents the hydrophilic surface from being hydrated for a desired period of time or temporarily limits the level of hydration for a desired period of time. In other words, the protective coating temporarily prevents or limits liquid water and/or water vapor from coming into contact with and being absorbed into the hydrophilic material. The protective coating limits/prevents hydration of a hydrophilic surface for a time period sufficient to allow the medical device to be radiation sterilized prior to hydration of the hydrophilic material. It is understood, without being held to any particular theory, that the protective coating coats the surface of the hydrophilic material and is absorbed into the interstices of the hydrophilic material's polymer matrix, thereby repelling the absorption of water into the hydrophilic coating, i.e., preventing or limiting the amount of liquid or vapor water from entering the hydrophilic polymer matrix. This causes a delay in the hydration of the hydrophilic material for a desired period of time, during which the medical device is exposed to sterilizing radiation. Eventually, liquid water or water vapor permeates through/past the protective coating and/or displaces at least some of the protective coating, thereby hydrating the hydrophilic material so that it is in a ready-to-use condition for the end user.

In one embodiment, the protective coating is a liquid that has a viscosity between about 30cP to about 60cP at 25°C. The protective coating may be applied in any suitable manner, such as by dip coating, spraying or painting. The amount or level of coating can vary depending on the viscosity of the protective coating, the dwell time, dip time or amount of protective coating applied by spraying or painting. The protective coating includes glycerol.

When the hydrophilic surface of the medical device is a hydrophilic coating, the hydrophilic coating may include a hydrophilic polymer, among other components, including but not limited to antioxidants, polyelectrolytes, plasticizers and the like. The hydrophilic polymer may be, for example, polyvinylpyrrolidone (PVP), polyethylene oxide, methyl cellulose, ethyl cellulose, polyethylene glycol, hydroxyl methylcellulose, hydroxypropyl cellulose, hydroxypropyl methylcellulose, carboxymethyl cellulose, polyvinyl alcohol, or mixtures thereof. Furthermore, when formed on the medical device, the hydrophilic coating may be a crosslinked hydrophilic coating.

In one method of forming a sterilized ready-to-use hydrophilically coated medical device assembly, a protective coating is applied over the hydrophilic surface, such as a hydrophilic coating, of the medical device by, for example, dipping the medical device into the protective coating. In one embodiment, the hydrophilic coating may be a crosslinked hydrophilic coating that was applied to the medical device in any suitable manner. In one embodiment, a hydrophilically coated urinary catheter may be dip coated with glycerol. The hydrophilically coated medical device is then placed within a hydration environment. For example, the medical device may be placed into a package along with liquid water and the package may then be sealed. In one embodiment, after the catheter is coated with glycerol, the catheter may be positioned in a sleeve that contains water and the sleeve may be the package or the sleeve may be placed in an outer package. The sleeve may be, for example, a no-touch sleeve wherein the user uses the sleeve to insert the catheter. The liquid water may be in direct contact with the medical device and/or the liquid water may produce a water vapor that is in contact with the medical device. The package may be made from a material that is liquid and gas impermeable. Furthermore, in one embodiment wherein the liquid water produces a water vapor for hydrating the hydrophilic coating, the liquid water is kept separated from the medical device. For instance, the liquid water may be retained by a liquid sequestering element, such as an absorbent member that holds the water or a compartment that is at least partially made from a liquid impermeable, gas permeable material.

After the medical device is placed in the hydration environment, the medical device is exposed to sterilization radiation. For example, after the medical device is placed in the package along with liquid water and the package is sealed, the package is exposed to sterilizing radiation, such as E-beam or gamma radiation. The distribution of the package is then delayed for a time sufficient for the hydration environment to hydrate the hydrophilic coating, i.e., sufficient amount of time to allow the liquid and/or vapor water to permeate through the protective coating to hydrate the device. In one embodiment, the packages may be stored for a period of time after radiation and then distributed.

## Claims

1. A method of sterilizing a medical device having a hydrophilic surface, comprising:
applying a protective coating comprising glycerol over a hydrophilic surface of a medical device, wherein the protective coating temporarily prevents hydration or limits the level of hydration of the hydrophilic surface;
placing the medical device in a hydration environment; and
exposing the medical device to sterilizing radiation, prior to the hydrophilic surface being hydrated into a ready-to-use condition.

2. The method of claim 1 wherein the hydrophilic surface comprises a hydrophilic coating disposed on the medical device.

3. The method of claim 2 wherein the hydrophilic coating comprises a crosslinked hydrophilic polymer.

4. The method of any one of the preceding claims wherein the hydrophilic surface comprises polyvinylpyrrolidone.

5. The method of any one of the preceding claims wherein the sterilizing radiation comprises E-beam or gamma radiation.

6. The method of any one of the preceding claims wherein the placing the medial device within a hydration environment comprises placing the catheter in a package containing a hydration fluid.

7. The method of claim 6 wherein the package is sealed prior to exposing the medical device to sterilizing radiation.

8. The method of any one of the preceding claims wherein the medical device is a urinary catheter.

9. A sterilized hydrophilically coated medical assembly, comprising:
a package containing:
a medical device having a hydrophilic coating thereon and a protective coating comprising glycerol over the hydrophilic coating, wherein the protective coating temporarily prevents hydration or limits the level of hydration of the hydrophilic surface; and
an amount of water within the package.

10. The assembly of claim 9 wherein the liquid water is in direct liquid contact with the medical device.

11. The assembly of claim 9 wherein the liquid water produces a water vapor that hydrates the medical device.

12. The assembly of claim 11 wherein the liquid water is separated from the medical device.

13. The assembly of any one claims wherein 9-12 the hydrophilic coating comprises polyvinylpyrrolidone.

14. The assembly of any one of claims 9-13 wherein the medical device is a urinary catheter.

## Patentansprüche

1. Verfahren zum Sterilisieren einer medizinischen Vorrichtung, die eine hydrophile Oberfläche aufweist, Folgendes umfassend:
Aufbringen einer Schutzbeschichtung, die Glycerin umfasst, auf eine hydrophile Oberfläche einer medizinischen Vorrichtung, wobei die Schutzbeschichtung vorübergehend eine Hydratation verhindert oder den Hydratationsgrad der hydrophilen Oberfläche begrenzt;
Platzieren der medizinischen Vorrichtung in einer Hydratationsumgebung; und
Aussetzen der medizinischen Vorrichtung einer Sterilisierungsstrahlung, bevor die hydrophile Oberfläche in einen gebrauchsfertigen Zustand hydratisiert wird.

2. Verfahren nach Anspruch 1, wobei die hydrophile Oberfläche eine hydrophile Beschichtung umfasst, die auf der medizinischen Vorrichtung angeordnet ist.

3. Verfahren nach Anspruch 2, wobei die hydrophile Beschichtung ein vernetztes hydrophiles Polymer umfasst.

4. Verfahren nach einem der vorhergehenden Ansprüche, wobei die hydrophile Oberfläche Polyvinylpyrrolidon umfasst.

5. Verfahren nach einem der vorhergehenden Ansprüche, wobei die Sterilisierungsstrahlung Elektronenstrahl- oder GammaStrahlung umfasst.

6. Verfahren nach einem der vorhergehenden Ansprüche, wobei das Platzieren der medizinischen Vorrichtung in einer Hydratationsumgebung ein Platzieren des Katheters in einer Packung umfasst, die Hydratationsfluid enthält.

7. Verfahren nach Anspruch 6,
wobei die Packung verschlossen wird, bevor die medizinische Vorrichtung einer Sterilisierungsstrahlung ausgesetzt wird.

8. Verfahren nach einem der vorhergehenden Ansprüche, wobei die medizinische Vorrichtung ein Harnkatheter ist.

9. Sterilisierte, hydrophil beschichtete medizinische Anordnung, Folgendes umfassend:
eine Packung, enthaltend:
eine medizinische Vorrichtung, die eine hydrophile Beschichtung darauf und eine Schutzbeschichtung, die Glycerin umfasst, über der hydrophilen Beschichtung aufweist, wobei die Schutzbeschichtung vorübergehend eine Hydratation verhindert oder den Hydratationsgrad der hydrophilen Oberfläche begrenzt; und
eine Menge Wasser in der Packung.

10. Anordnung nach Anspruch 9, wobei das flüssige Wasser in direktem Flüssigkeitskontakt mit der medizinischen Vorrichtung ist.

11. Anordnung nach Anspruch 9, wobei das flüssige Wasser einen Wasserdampf erzeugt, der die medizinische Vorrichtung hydratisiert.

12. Anordnung nach Anspruch 11, wobei das flüssige Wasser von der medizinischen Vorrichtung getrennt ist.

13. Anordnung nach einem der Ansprüche 9-12, wobei die hydrophile Beschichtung Polyvinylpyrrolidon umfasst.

14. Anordnung nach einem der Ansprüche 9-13, wobei die medizinische Vorrichtung ein Harnkatheter ist.

## Revendications

1. Procédé de stérilisation d'un dispositif médical ayant une surface hydrophile, comprenant :
l'application d'un revêtement protecteur comprenant du glycérol sur une surface hydrophile d'un dispositif médical, dans lequel le revêtement protecteur empêche l'hydratation ou limite le niveau d'hydratation de la surface hydrophile temporairement ;
le placement du dispositif médical dans un environnement d'hydratation ; et
l'exposition du dispositif médical à un rayonnement stérilisant, avant que la surface hydrophile ne soit hydratée dans une condition prête à l'utilisation.

2. Procédé selon la revendication 1 dans lequel la surface hydrophile comprend un revêtement hydrophile disposé sur le dispositif médical.

3. Procédé selon la revendication 2 dans lequel le revêtement hydrophile comprend un polymère hydrophile réticulé.

4. Procédé selon l'une quelconque des revendications précédentes dans lequel la surface hydrophile comprend de la polyvinylpyrrolidone.

5. Procédé selon l'une quelconque des revendications précédentes dans lequel le rayonnement stérilisant comprend un faisceau E ou un rayonnement gamma.

6. Procédé selon l'une quelconque des revendications précédentes dans lequel le placement du dispositif médical à l'intérieur d'un environnement d'hydratation comprend le placement du cathéter dans un emballage contenant un fluide hydratation.

7. Procédé selon la revendication 6 dans lequel l'emballage est scellé avant l'exposition du dispositif médical à un rayonnement stérilisant.

8. Procédé selon l'une quelconque des revendications précédentes dans lequel le dispositif médical est un cathéter urinaire.

9. Ensemble médical à revêtement hydrophile stérilisé, comprenant :
un emballage contenant :
un dispositif médical ayant un revêtement hydrophile sur celui-ci et un revêtement protecteur comprenant du glycérol sur le revêtement hydrophile, dans lequel le revêtement protecteur empêche l'hydratation ou limite le niveau d'hydratation de la surface hydrophile temporairement ; et
une quantité d'eau à l'intérieur de l'emballage.

10. Ensemble selon la revendication 9 dans lequel l'eau liquide est en contact liquide direct avec le dispositif médical.

11. Ensemble selon la revendication 9 dans lequel l'eau liquide produit une vapeur d'eau qui hydrate le dispositif médical.

12. Ensemble selon la revendication 11 dans lequel l'eau liquide est séparée du dispositif médical.

13. Ensemble selon l'une quelconque des revendications 9 à 12 dans lequel le revêtement hydrophile comprend de la polyvinylpyrrolidone.

14. Ensemble selon une quelconque des revendications 9 à 13 dans lequel le dispositif médical est un cathéter urinaire.
